# EUROPÄISCHE PATENTSCHRIFT

(11) **EP 1 027 880 B1**
(45) Veröffentlichungstag und Bekanntmachung des Hinweises auf die Patenterteilung: **13.07.2005**
(21) Anmeldenummer: 00101361.4
(22) Anmeldetag: 25.01.2000
(51) Int. Cl.: A61K 7/32

(54) **Verwendung von quaternären Ammoniumverbindungen als Antitranspirantien**
Use of quaternary ammonium compounds as antiperspirants
Utilisation de composés d'ammonium quaternaire comme antitranspirants

(30) Priorität: 11.02.1999 DE 19905585
(43) Veröffentlichungstag der Anmeldung: 16.08.2000
(73) Patentinhaber: Beiersdorf Aktiengesellschaft, 20245 Hamburg (DE); Bode Chemie GmbH & Co., 22525 Hamburg (DE)
(72) Erfinder: Wolf, Florian, Dr., 20251 Hamburg (DE); Keskin, Maike, Dr., 22765 Hamburg (DE); Knieler, Roland, Dr., 22529 Hamburg (DE); Pietsch, Hanns, Dr., 20148 Hamburg (DE)

(56) Entgegenhaltungen:
- DE-A- 2 004 872
- GB-A- 656 748
- US-A- 4 125 600
- US-A- 4 524 062

## Beschreibung

Die vorliegende Erfindung betrifft die Verwendung an sich bekannter Substanzen als antitranspirierend wirksame Agentien. Insbesondere betrifft die Erfindung die Verwendung dieser Stoffe in kosmetischen oder dermatologischen Zubereitungen.

Körpergerüche in ihrem ursprünglichen Sinn dienen der sozialen Kommunikation. Beispielsweise haben spezielle, von den Duftdrüsen abgesonderte Stoffe (Pheromone) in der Tierwelt einen wesentlichen Einfluß auf Sexual-, Territorial- und Aggressionsverhalten oder sind Ausdruck von Individualität bzw. Rangordnung. Für den Menschen hat diese Art von Verständigung jedoch sehr stark an Bedeutung verloren, wie die vergleichsweise kümmerliche Leistungsfähigkeit des menschlichen Geruchssinns andeutet. Körpergeruch wird in modernen, westlich geprägten Industriegesellschaften als aufdringlich, abstoßend und ungepflegt angesehen. Die Anwendung von kosmetischen oder dermatologischen Desodorantien bietet eine Möglichkeit, unangenehmem Körpergeruch entgegenzuwirken.

Körpergeruch entsteht, wenn der an sich geruchlose frische Schweiß durch Mikroorganismen zersetzt wird. Die grampositiven Bakterien bauen das Sebum, Eiweißstoffe und die gelösten Bestandteile des Schweißes ab. Dabei werden geruchsintensive kurzkettige Fettsäuren und Amine frei. Insbesondere der apokrine Schweiß transportiert auf seinem Weg über die Talgdrüsen und Haarfollikel eine Reihe von Mikroorganismen an die Hautberfläche, die recht bald in die Zersetzung eingreifen. Auf der Haut befindet sich aber auch eine bestimmte residente Mikroorganismenpopulation, welche, ergänzt durch transiente Mikrorganismen aus der Umwelt, den Abbau des Schweißes beeinflußt.

Den üblichen kosmetischen Desodorantien liegen unterschiedliche Wirkprinzipien zugrunde.

Übliche in Desodorantien eingesetzte Wirkstoffe besitzen beispielsweise antimikrobielle Eigenschaften, die die mikrobielle Zersetzung des frischen Schweißes verhindern. Eine desodorierende Wirkung läßt sich aber auch durch enzyminhibierende, aber nicht antimikrobiell wirkende Substanzen und/oder durch Ionenaustauscher erreichen. Kationenaustauscher z. B. sollen Ammoniak, Indol und ähnliche Gerüche absorbieren. Wirkstoffe mit antitranspiranten Eigenschaften hingegen sind solche, die das Schwitzen des menschlichen Körpers reduzieren oder ganz unterdrücken, wobei die Wirkung bei topischer Applikation in der Regel lokal ist.

Die in sogenannten Antitranspirantien eingesetzten Wirkstoffe hemmen in erster Linie die Schweißsekretion, haben aber im allgemeinen auch eine gewisse antibakterielle Wirkung. Sie wirken blockierend auf den Teil des Ausführungsganges der Schweißdrüsen, der durch das Stratum comeum führt Sie verengen bzw. verschließen die Schweißdrüsen, so daß das Schwitzen ganz oder teilweise unterdrückt wird.

Als antitranspirante Wirkstoffe sind Aluminiumsalze seit langem bekannt. Aluminiumchlorid, Aluminiumchlorhydrat, -nitrat, -sulfat, -acetat usw. werden seit einigen Jahren als anti-transpirante oder antiperspirante Wirkstoffe verwendet. Daneben finden auch Zink-, Magnesium- und Zirkoniumverbindungen Anwendung. Der Stand der Technik wird beispielsweise beschrieben in: *H. P. Fiedler, Der Schweiß, Editio Cantor, Aulendorf, 2. Auflage, S*. *303-377, Kapitel K:* "*Mittel zur Hemmung der Transpiration"*. Von den dort als Antitranspirant-Wirkstoffe beschriebenen Metallverbindungen haben sich für die Anwendung in kosmetischen und dermatologischen Antitranspirantien nur die Aluminiumsalze und - in etwas geringerem Maße - die Aluminium/Zirkoniumsalze durchgesetzt.

Der Hauptnachteil der sehr wirksamen Aluminiumchloridlösungen ist, daß sie stark sauer reagieren und daher mitunter ein Brennen auf der Haut und eine Rötung verursachen können, welche eventuell mit Abschilferung verbunden sein kann. Aus diesem Grund setzt man üblicherweise die teilneutralisierten, besser hautverträglichen, aber nicht ganz so wirksamen Aluminiumhydroxychloride ein, die auch einen etwas günstigeren pH-Wert von etwa 4 aufweisen. Allerdings können auch diese bei häufiger Anwendung und bei empfindlichen Personen Hautschäden hervorrufen oder zu unerwünschten Verfärbungen auf Textilien führen. Letzteres kann beispielsweise durch die Verwendung von Zirkonium/Aluminium-Mischsalzen verringert werden.

Antitranspirantien, welche Aluminiumhydroxychloride als Wirkstoffe enthalten, werden vor allem in Form von wäßrigen Lösungen und als Pulver oder Granulate (Trockensprays) eingesetzt. Auch andere Zubereitungsformen, wie z. B. alkoholische Lösungen, Stiftformulierungen, Emulsionen, Mikroemulsionen, Cremes, Salben, Roller und dergleichen, sind prinzipiell denkbar, allerdings teilweise schwierig stabil zu formulieren.

Für Deostifte auf Basis von Natriumstearat oder anderen Seifen beispielsweise sind die sauren Aluminiumsalze nicht geeignet, da sie zum Aussalzen der Seife und somit zu chemischer Unverträglichkeit führen. Bei größeren Zusatzmengen, die für Antitranspirant-Stifte notwendig sind, wird außerdem die Bildung eines festen Seifengels verhindert.

Sollen kosmetische oder pharmazeutische Zubereitungen bestimmte Wirkstoffe enthalten, ist es prinzipiell immer denkbar, daß die übrigen Bestandteile mit den Wirkstoffen nicht kompatibel sind. Aus diesen und aus Gründen der Verträglichkeit ist es daher stets zu bevorzugen, die Einsatzkonzentrationen der Wirkstoffe möglichst niedrig zu halten, selbst bei Verwendung an sich unbedenklicher Substanzen.

Aufgabe der Erfindung war es daher, den Nachteilen des Standes der Technik abzuhelfen und antitranspirante Wirkstoffe zu finden, welche eine gute Wirkung zeigen, nicht so stark sauer sind, eine gute Hautverträglichkeit aufweisen und Textilien nicht schädigen oder verfärben.

Eine weitere Aufgabe der vorliegenden Erfindung war, Zubereitungen zu entwickeln, welche als Grundlage für kosmetische oder dermatologische Desodorantien bzw. Antitranspirantien geeignet sind und sich durch gute Hautverträglichkeit auszeichnen.

Es war indes überraschend und für den Fachmann in keiner Weise vorhersehbar, daß die Verwendung von quaternären Ammoniumverbindungen gewählt aus der Gruppe, welche gebildet wird aus quaternären Ammoniumverbindungen der allgemeinen Strukturformel 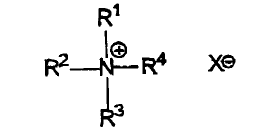 und Alkylpyridiniumsalzen der allgemeinen Strukturformel 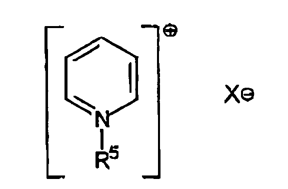 als antitranspirierend wirksame Agentien die Nachteile des Standes der Technik beseitigt.

R¹ und R² stellen unabhängig voneinander Methyl- oder Ethylgruppen dar, R³ wird gewählt aus der Gruppe der Alkylreste mit 1 bis 18 Kohlenstoffatomen, R⁴ wird gewählt aus der Gruppe, welche gebildet wird aus den Alkylresten mit 8 bis 18 Kohlenstoffatomen und den Aralkylresten mit 7 bis 18 Kohlenstoffatomen, und R⁵ wird gewählt aus der Gruppe der Alkylreste mit 10 bis 18 Kohlenstoffatomen.

X⁻ stellt ein anorganisches oder organisches Anion dar, beispielsweise ein Halogenid (z. B. Chlorid oder Bromid), ferner ein anorganisches Oxo-Element-Anion (von diesen insbesondere Sulfat, Carbonat, Phosphat, Borat und Aluminat) sowie ein Alkylsulfat (insbesondere Ethylsulfat) oder z. B. ein Lactat, Acetat, Benzoat, Propionat, Tartrat, Citrat und andere mehr.

Bevorzugt im Sinne der vorliegenden Erfindung sind quaternäre Ammoniumverbindungen, in denen die Reste R¹, R², R³ und R⁴ rein aliphatische Verbindungen darstellen und X⁻ kein Halogenid ist

Quaternäre Ammoniumverbindungen sind hancielsübliche Produkte und werden z. B. als bakterizide und fungizide Wirkstoffe zur Herstellung von Desinfektionsmitteln eingesetzt, sie sind oberflächenaktiv und werden als Netzmittel oder Emulgierhilfsmittel verwendet sowie im Bereich der Textilindustrie zur Avivage (= Griffigmachen) von Textilien.

Auch die Verwendung von quaternären Ammoniumverbindungen in kosmetischen oder dermatologischen Zubereitungen ist an sich bekannt. Sie werden bevorzugt zur Herstellung konditionierend wirkender Haarpflegemittel verwendet. Wesentliche Eigenschaften von Haarpflegemitteln, wie beispielsweise die Verbesserung von Kämmbarkeit und Griff sowie die Verhinderung statischer Aufladung der Haare werden durch den Einsatz von quaternären Ammoniumverbindungen verbessert.

Zwar werden in *H. P. Fiedler, Der Schweiß (Editio Cantor, Aulendorf, 2. Auflage, S*. *358 ff, "Physiologische Antiperspirants")* auch einige spezielle quatemäre Ammoniumverbindungen als physiologisch wirksame Antitranspirantien genannt. Allerdings werden diese nicht topisch angewendet, sondern auf iontophoretischen Wege in die Haut eingeschleust (d. h. unter dem Einfluß des elektrischen Stromes durch die Haut in den Körper gebracht), und ferner haben diese Verbindungen entweder nur kurzkettige Reste R¹, R², R³ und R⁴ (Methyl-, Ethyl- und/oder Butyl-) oder zusätzlich solche, die eine OH-Gruppe tragen (wie beispielsweise das dort genannte Butyldimethyl(benzoylethanol)-ammoniumbromid). Nach der a. o. O. dargestellten Tabelle zeigen die untersuchten quaternären Ammoniumverbindungen zudem völlig unterschiedliche Wirksamkeiten, so daß der Stand der Technik keinerlei Hinweise geben konnte, welche Rückschlüsse auf die erfindungsgemäße Verwendung erlaubt hätten.

Erfindungsgemäß bevorzugt ist die Verwendung von Benzalkoniumchlorid (N-Alkyl-N,N-dimethylbenzylammoniumchlorid), Cetrimoniumbromid (N-Hexadecyl-N,N,N-trimethylammoniumbromid), Cetyltrimethylammoniumbromid, Mecetroniumethylsulfat (N-Hexadecyl-N-ethyl-N,N-dimethylammoniumethylsulfat), N,N-Didecyl-N,N-dimethylammoniumchlorid und/oder N,N-Dioctyl-N,N-dimethylammoniumchlorid. Ferner vorteilhaft ist die Verwendung von Substanzen gewählt aus der Gruppe der Benzyltrialkylammoniumchloride oder -bromide, wie beispielsweise Benzyldimethylstearylammoniumchlorid, als antitranspirierend wirksame Agentien.

Erfindungsgemäß bevorzugt ist ferner die Verwendung von Lauryl- oder Cetylpyrimidiniumchlorid als antitranspirierend wirksame Agentien.

Ganz besonders bevorzugt ist die erfindungsgemäße Verwendung von Mecetroniumethylsulfat. Dieses zeichnet sich zudem insbesondere durch eine gute Hautverträglichkeit aus.

Die Wirkstoffe können erfindungsgemäß vorteilhaft sowohl einzeln als auch im Gemisch vorliegen.

Entsprechend der erfindungsgemäßen Verwendung sind die Zubereitungen besonders vorteilhaft dadurch gekennzeichnet, daß der oder die erfindungsgemäßen antitranspiranten Wirkstoffe in Konzentrationen von 0,01 bis 20,00 Gew.-%, bevorzugt 0,1 bis 10,00 Gew.-%, jeweils bezogen auf das Gesamtgewicht der Zusammensetzung, vorliegt oder vorliegen.

Besonders vorteilhaft sind einfache alkoholische und wäßrige Lösungen, beispielsweise in Ethanol, Propanol-1 sowie Propanol-2, insbesondere solche, welche eine Wirkstoffkonzentration von mehr 0,1 Gew.-%, bezogen auf das Gesamtgewicht der Zusammensetzung, aufweisen. Ebenfalls vorteilhaft ist die erfindungsgemäße Verwendung der Wirkstoffe in Emulsionen und Cremes, welche mindestens eine Fettphase enthalten, wobei die Konzentration der Wirkstoffe in diesen Zubereitungen vorteilhaft aus dem Bereich zwischen 2 und 5 Gew.-%, bezogen auf das Gesamtgewicht der Zubereitungen, gewählt wird.

Stellen die erfindungsgemäß erhaltenen Zubereitungen Desodorantien/Antitranspirantien dar, so können zusätzlich zu den erfindungsgemäß verwendeten quaternären Ammoniumverbindungen alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäß erhaltenen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quatemäre Ammoniumverbindungen, Nelkenöl, Minzöl, Thymianöl, Triethylcitrat, Famesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Auch die Verwendung weiterer üblicher Antitranspiranswirkstoffe kann vorteilhaft im Sinne der vorliegenden Erfindung sein, insbesondere die Verwendung von Adstringentien, beispielsweise von basischen Aluminiumchloriden in erfindungsgemäßen, antitranspirierend wirksamen Zubereitungen.

Entsprechend der erfindungsgemäßen Verwendung können die kosmetischen Zubereitungen als Desodorantien bzw. Antitranspirantien bezeichnet werden. Vorteilhaft können sie beispielsweise in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O-oder O/W-Emulsionen, z.B. Crèmes oder Lotionen. Weiterhin können die kosmetischen Desodorantien vorteilhaft in Form von desodorierenden bzw. antitranspirierend wirkenden Tinkturen, desodorierenden bzw. antitranspirierend wirkenden Intimreinigungsmitteln, desodorierenden bzw. antitranspirierend wirkenden Pudern oder deodorierenden bzw. antitranspirierend wirkenden Pudersprays vorliegen.

Als Treibmittel für aus Aerosolbehältem versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Als übliche kosmetische Trägerstoffe zur Herstellung der deodorierenden bzw. antitranspirierend wirkenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z. B. Hydroxyethyl- oder Hydroxypropylcellulose, polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothiaglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Di-Iaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Ubichinon und Ubichinol und deren Derivate, Vitamin C und Derivate (z.B. Ascorbylpalmitat. Mg-Ascorbylphosphat, Ascorbylacetat), Tocopherole und Derivate (z.B. VitaminE-acetat), sowie Koniferylbenzoat des Benzoäharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononyistearat Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzost, CaPryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Emulsionen entsprechend der vorliegenden Erfindungen enthalten einen oder mehrere Emulgatoren, insbesondere vorteilhaft gewählt aus der Gruppe Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylenglycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; lsostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-lsostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphasphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat, Acrylat/C₁₀₋₃₀-Alkylacrylat-Crosspolymer, Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fett säureester.

Obliche Grundstoffe, welche für die Verwendung als kosmetische Stifte im Sinne der vor liegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Ver dickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Zuberereitungen gemäß der vorliegenden Erfindung können sich auch durch einen Gehalt an Tensiden auszeichnen.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfemung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quartemären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroyisarcosinat und Natriumcocoylsarkosinat,
4. Taurate, beispielsweise Natriumlauroyltaurat und Natriummethylcocoyltaurat,
5. Alaninate
Carbonsäuren und Derivate, wie
1. Carbonsäuren, beispielsweise Laurinsäure, Aluminiumstearat, Magnesiumalkanolat und Zinkundecylenat, und Derivate, wie z. B. Acyllactylate, Lauroyllactylat, Caproyllactylat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Zubereitungen gemäß der vorliegenden Erfindung können ferner vorteilhaft weitere quatemäre Ammoniumverbindungen enthalten, insbesonder solche, welche als quatemäre Tenside bezeichnet werden. Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft zu verwenden sind Alkylbetain, Alkylamidopropylbetain und Alkylamidopropylhydroxysulfain, Alkylamidethyltrimethylammoniumethersulfate, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat,Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Der Nachweis der Wirksamkeit wurde an menschlichen Probanden mit Hilfe der sogenannten "Imprintmethode" geführt Bei dieser Methode wird das zu untersuchende Material am Unterarm innen mehrere Tage hintereinander (topisch) angewendet, sodann begeben sich die Probanden in die Sauna zum Schwitzen. An der Untersuchungsstelle wird eine Silikonmasse aufgetragen, die während der Schwitzphase aushärtet. An den Stellen, an denen Schweisströpfchen austreten, bilden sich Abdrücke in der Silikonmasse, welche bildanalytisch ausgewertet werden können. Nach diesem Verfahren wurden zunächst die Wirkstoffe in wäßriger Lösung untersucht und sodann entsprechende kosmetische Zubereitungen.

### Beispiele:

### Beispiele 1 - 3

Es wurden wässrige Lösungen aus Mecetroniumethylsulfat (MES) hergestellt und an 15 Probanden die Schweissreduktion mit Hilfe der Imprint-Methode am inneren Unterarm geprüft. Zum Vergleich wurde eine 10 %ige Aluminiumchlorhydrat-Lösung verwendet

Ergebnisse:

| | |
|---|---|
| 10% Aluminiumchlorhydrat | 93 % Reduktion der Schweißsekretion |
| 1% MES Lösung | 93 % Reduktion der Schweißsekretion |
| 3%MES Lösung | 95 % Reduktion der Schweißsekretion |
| 5% MES Lösung | 95 % Reduktion der Schweißsekretion |

### Beispiel 4

Es wurde ein antitranspirantes Händedesinfektionsmittel hergestellt, bestehend aus:

| **Gew.-%** | |
|---|---|
| 0,2 | MES |
| 30,0 | Propanol-1 |
| 45,0 | Propanol-2 |
| 1,0 | Tetradecanol |
| 0,5 | Glycerol |
| q.s. | Parfüm |
| q.s. | Farbstoff |
| ad 100 | entmineralisiertes Wasser |

Mit diesem Präparat haben sich 15 Probanden die Hände und den Unterarm während drei Minuten eingerieben, entsprechend einer chirurgischen Händedesinfektion, derart, daß Hände und Unterarme 3 Minuten feucht gehalten wurden. Nach dem Abdunsten des Präparates wurden OP-Handschuhe aus Naturkautschuk-Latex übergezogen und drei Stunden getragen, danach ausgezogen und die Schweißmenge durch Abspülen der Hände und Handschuhe mit getrocknetem Aceton und anschliessender Karl-Fischer-Titration besimmt.

Das gleiche Verfahren wurde mit einem Vergleichsprodukt ohne Mecetroniumethylsulfat durchgeführt. Außerdem wurde beurteilt, ob Hautreizungen oder allergische Reaktionen auftraten.

### Ergebnis:

Das MES-haltige Produkt ergab eine ca. 22 % geringere Schweissmenge nach drei Stunden Handschuhtragen im Verhältnis zum Vergleichsprodukt.
Keine Hautreaktionen.

### Beispiel 5

Es wurde ein antitranspirantes Händedesinfektionsmittel hergestellt, bestehend aus:

| **Gew.-%** | |
|---|---|
| 0,100 | MES |
| 0,100 | Caprylsäure/Caprinsäuretriglycerid |
| 0,100 | Glycerol |
| 1,000 | Butanon |
| 3,700 | Wasser, entionisiert |
| 95,000 | Ethanol (absolut) |

Dieses Präparat wurde einem Beispiel 4 analogen Vergleichstest unterzogen.

Ergebnis:
Das MES-haltige Podukt ergab gegenüber dem MES-freien Produkt eine Schweißn=duk tion von ca. 17 %.
Keine Hautreaktionen.

### Beispiel 6

Es wurde ein alkoholisches antitranspirantes Fussspray hergestellt aus:

| **Gew.-%** | |
|---|---|
| 10,000 | MES |
| 55,000 | Ethanol (absolut) |
| 34,480 | Wasser(entionisiert) |
| 0,250 | Fettalkoholpolyglykolether(PPG-5-Laureth-5) |
| 0,100 | Menthol |
| 0,100 | Parfüm |
| 0,050 | Kokosfettsäurediethanolamid |
| 0,020 | Allantoin |

Nach der beschriebenen Imprint-Methode wurde eine Schweißreduktion von ca. 10 % ermittelt.
Keine Hautreaktionen.

### Beispiel 7

Es wurde eine antitranspirante Mikroemulsion hergestellt aus:

| Gew.-% | |
|---|---|
| 75,750 | Demineralisiertes Wasser |
| 8,000 | Mecetroniumethylsulfat |
| 3,500 | Capryl-/Caprinsäureester ungesättigter Fettalkohole |
| 3,500 | Di-n-octylether |
| 3,000 | 1,2-Pentandiol |
| 2,100 | Glycerolmonostearat |
| 0,280 | Stearylalkoholpolyethylenglycolether (12 EO) |
| 0,245 | Cetyl/Stearylalkohol, |
| 0,175, | Cetylpalmitat |
| 1,700 | Cetyl/Stearylalkoholpolyethylenglycolether (20 EO) |
| 0,750 | Panthenol |
| 0,500 | Nichtionisches, hydrophiles Bienenwachs |
| 0,300 | Parfüm |
| 0,176 | Glycrolmonodecanoat |
| 0,014 | Glycerol |
| 0,010 | Glyceroldidecanoat |

Nach der beschriebenen Imprint-Methode wurde eine Schweißreduktion von ca. 10 % ermittelt.
Keine Hautreaktionen.

### Beispiel 8

Es wurde eine antitranspirante und antimikrobielle Fußcreme herestellt aus:

| Gew.-% | |
|---|---|
| 2,00 | Mecetroniumethylsulfat |
| 4,00 | Glycerol |
| 9,15 | Tetradecanol |
| 86,65 | Demineralisiertes Wasser |

Nach der beschriebenen Imprint-Methode wurde eine Schweißreduktion von ca. 10 % ermittelt.
Keine Hautreaktionen

### Beispiel 9

Es wurde ein antitranspiranter Spray hergestellt aus:

| Gew.-% | |
|---|---|
| 0,70 | Mecetroniumetylsulfat |
| 1,00 | Dexpanthenol |
| 0,50 | Benzoesäure |
| 0,10 | Parfüm |
| 97,70 | Demineralisiertes Wasser |

### Beispiel 10

Es wurde ein antitranspiranter Roll-on-Stift hergestellt, bestehend aus:

| Gew.-% | |
|---|---|
| 1,00 | Mecetroniumethylsulfat |
| 1,00 | Triethylcitrat |
| 0,50 | Allantoin |
| 0,10 | Parfüm |
| 0,05 | Citronensäure |
| 97,35 | Demineralisiertes Wasser |

### Beispiel 11

Es wurde ein antitranspirantes Spray hergestellt, bestehend aus:

| Gew.-% | |
|---|---|
| 3,00% | Mecetroniumethylsulfat |
| 15,00% | Ethanol |
| 1,00% | Dexpanthenol |
| 81,00% | Demineralisiertes Wasser |

## Patentansprüche

1. Verwendung von quaternären Ammoniumverbindungen gewählt aus der Gruppe, welche gebildet wird aus quaternären Ammoniumverbindungen der allgemeinen Strukturformel 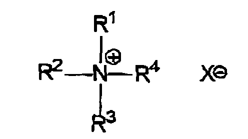 und Alkylpyridiniumsalzen der allgemeinen Strukturformel 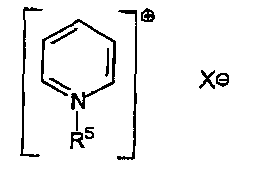 als antitranspirierend wirksame Agentien, wobei R¹ und R² unabhängig voneinander Methyl- oder Ethylgruppen darstellen, R³ gewählt wird aus der Gruppe der Alkylreste mit 1 bis 18 Kohlenstoffatomen, R⁴ gewählt wird aus der Gruppe, welche gebildet wird aus den Alkylresten mit 8 bis 18 Kohlenstoffatomen und den Aralkylresten mit 7 bis 18 Kohlenstoffatomen, R⁵ wird gewählt aus der Gruppe der Alkylreste mit 10 bis 18 Kohlenstoffatomen und worin X⁻ ein anorganisches oder organisches Anion darstellt.

2. Verwendung nach Anspruch 1, **dadurch gekennzeichnet, daß** der oder die antitranspirierend wirksamen Agentien gewählt werden aus der Gruppe Decyldimethylethylammoniumethylsulfat, Dodecyldimethylethylammoniumethylsulfat und Tetradecyfdimethylethylammoniumethylsulfat.

3. Verwendung nach Anspruch 1, **dadurch gekennzeichnet, daß** die quaternäre Ammoniumverbindung oder die quaternären Ammoniumverbindungen in Konzentrationen von 0,01 bis 20,00 Gew.-%, bevorzugt 0,1 bis 10,00 Gew.-% vorliegt oder vorliegen, jeweils bezogen auf das Gesamtgewicht der Zusammensetzung.

4. Verwendung nach Anspruch 1, **dadurch gekennzeichnet, daß** weitere Antitranspiranswirkstoffe zugegen sind.

## Claims

1. Use of quaternary ammonium compounds chosen from the group which is formed from quaternary ammonium compounds of the general structural formula 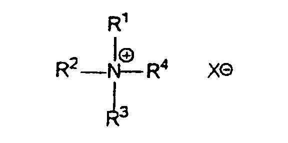 and alkylpyridinium salts of the general structural formula 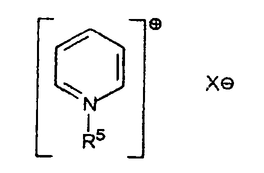 as antiperspirant agents, where R¹ and R², independently of one another, are methyl or ethyl groups, R³ is chosen from the group of alkyl radicals having 1 to 18 carbon atoms, R⁴ is chosen from the group which is formed from the alkyl radicals having 8 to 18 carbon atoms and the aralkyl radicals having 7 to 18 carbon atoms, R⁵ is chosen from the group of alkyl radicals having 10 to 18 carbon atoms and in which X⁻ is an inorganic or organic anion.

2. Use according to Claim 1, **characterized in that** the antiperspirant agent or agents are chosen from the group consisting of decyldimethylethylammonium ethylsulphate, dodecyldimethylethylammonium ethylsulphate and tetradecyldimethylethylammonium ethylsulphate.

3. Use according to Claim 1, **characterized in that** the quaternary ammonium compound or the quaternary ammonium compounds is or are present in concentrations of from 0.01 to 20.00% by weight, preferably 0.1 to 10.00% by weight, in each case based on the total weight of the composition.

4. Use according to Claim 1, **characterized in that** further antiperspirant active ingredients are present.

## Revendications

1. Utilisation de composés ammonium quaternaires choisis parmi le groupe constitué de composés ammonium quaternaires de formule structurale générale 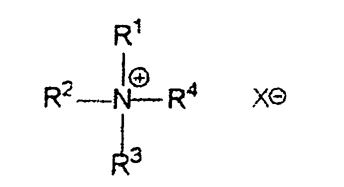 et de sels d'alkylpyridinium de formule structurale générale 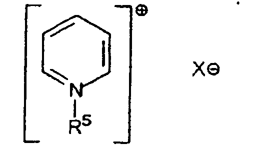 en tant qu'agents à action anti-transpirante, dans lesquelles R¹ et R² représentent, indépendamment l'un de l'autre des groupes méthyle ou éthyle, R³ est choisi parmi le groupe des radicaux alkyle ayant de 1 à 18 atomes de carbone, R⁴ est choisi parmi le groupe constitué des radicaux alkyle ayant de 8 à 18 atomes de carbone et des radicaux aralkyle ayant de 7 à 18 atomes de carbone, R⁵ est choisi parmi le groupe des radicaux alkyle ayant de 10 à 18 atomes de carbone, et où X⁻ représente un anion inorganique ou organique.

2. Utilisation selon la revendication 1, **caractérisée en ce que** le ou les agent(s) à action anti-transpirante est/sont choisi(s) parmi le groupe constitué de l'éthylsulfate du décyldiméthyléthylammonium, de l'éthylsulfate du dodécyldiméthyléthylammonium et de l'éthylsulfate du tétradécyldiméthyléthylammonium.

3. Utilisation selon la revendication 1, **caractérisée en ce que** le composé ammonium quaternaire ou les composés ammonium quaternaires est/sont présent (s) en concentration de 0,01 à 20,00 % en poids, de préférence de 0,1 à 10,00 % en poids dans chaque cas par rapport au poids total de la composition.

4. Utilisation selon la revendication 1, **caractérisée en ce que** des substances actives anti-transpirantes supplémentaires sont présentes.
